(19) **Europäisches Patentamt European Patent Office Office européen des brevets**

(11) **EP 4 251 556 B1**

(12) **EUROPEAN PATENT SPECIFICATION**

| | |
|---|---|
| (45) Date of publication and mention of the grant of the patent: **28.08.2024 Bulletin 2024/35** | (51) International Patent Classification (IPC): ***B67D 1/07*** *(2006.01)* ***A61L 2/10*** *(2006.01)* ***B67D 1/08*** *(2006.01)* ***C02F 1/32*** *(2023.01)* |
| (21) Application number: **21823778.2** | (52) Cooperative Patent Classification (CPC): **B67D 1/07; A61L 2/10; B67D 1/0879; B67D 1/0887; B67D 1/0888; C02F 1/32; C02F 9/20;** A61L 2/18; A61L 2/20; B67D 2001/075; B67D 2210/00015; C02F 1/001; C02F 1/76; C02F 1/78; C02F 2307/02; (Cont.) |
| (22) Date of filing: **24.11.2021** | |
| | (86) International application number: **PCT/EP2021/082826** |
| | (87) International publication number: **WO 2022/112332 (02.06.2022 Gazette 2022/22)** |

(54) **LIQUID DISPENSER WITH DISPENSING CHAMBER HAVING AN UV SOURCE**

FLÜSSIGKEITSPENDER MIT ABGABEKAMMER MIT UV QUELLE

DISTRIBUTEUR DE LIQUIDE AVEC CHAMBRE DE DISTRIBUTION AVEC SOURCE UV

| | |
|---|---|
| (84) Designated Contracting States: **AL AT BE BG CH CY CZ DE DK EE ES FI FR GB GR HR HU IE IS IT LI LT LU LV MC MK MT NL NO PL PT RO RS SE SI SK SM TR** | • **KHAKI, Amir Houshang Southampton, Hampshire SO17 1XQ (GB)** |
| (30) Priority: **24.11.2020 GB 202018488** | (74) Representative: **Bridle Intellectual Property 6F Thomas Way Lakesview International Business Park Canterbury, Kent CT3 4JZ (GB)** |
| (43) Date of publication of application: **04.10.2023 Bulletin 2023/40** | (56) References cited: **EP-A1- 3 220 366   CN-A- 107 773 041 GB-A- 2 460 957   US-A- 4 867 052 US-A1- 2007 137 726   US-A1- 2011 100 865** |
| (73) Proprietor: **Chamberlains Aqua Systems Limited Southampton Hampshire SO17 1XQ (GB)** | |
| (72) Inventors: • **SADR, Seyed Mohammad Kazem Southampton, Hampshire SO17 1XQ (GB)** | |

(52) Cooperative Patent Classification (CPC): (Cont.)
C02F 2307/10

## Description

The present invention relates to a liquid dispenser and, in particular, to a liquid dispenser for dispensing water in a safe manner, more particularly a hands-free liquid dispenser which minimises cross-contamination by infectious microbial agents.

In this context, the term infectious microbial agent includes bacteria, viruses and fungi.

Conventional water dispensers require a user to contact the dispenser, which gives rise to the potential for infectious agents, such as bacteria or viral particles, to be transferred to a surface of the dispenser. Even if the dispenser is capable of being operated in a "hands-free" way, there is still the possibility that surfaces of the dispenser may become contaminated with infectious agents. This then poses an infection risk to subsequent users.

According to a first aspect of the invention, there is provided a liquid dispenser comprising a dispensing chamber, wherein the dispensing chamber includes a liquid dispensing apparatus, wherein the liquid dispensing apparatus includes a dispensing port; a closable door which has an open configuration in which access to the chamber is permitted, and a closed configuration in which access to the chamber is prevented; and a disinfection system comprising an ultra violet light source, wherein the chamber is disinfected via the disinfection system when the door is in its closed configuration.

Thus, the dispensing chamber of the invention can be closed by the door when not in use. This prevents infectious agents from entering the dispensing chamber. Furthermore, when the door is closed, the dispensing chamber may be disinfected by the disinfection system. Such an arrangement helps to maintain a sterile environment within the dispensing chamber.

In one embodiment, a liquid container, for example a cup, a bottle or flask, is located within the chamber, wherein the liquid container is disinfected via the disinfection system when the door is in its closed configuration.

The electromagnetic spectrum of ultraviolet radiation (UVR), defined most broadly as 10-400 nanometres, can be subdivided into a number of ranges recommended by the ISO standard

| Name | Abbreviation | Wavelength (nm) | Photon energy (eV, aJ) |
|---|---|---|---|
| Ultraviolet C | UVC | 100-280 | 4.43-12.4, 0.710-1.987 |
| Ultraviolet B | UVB | 280-315 | 3.94-4.43, 0.631-0.710 |
| Ultraviolet A | UVA | 315-400 | 3.10-3.94, 0.497-0.631 |

Ultra violet (UV) light, in particular UVC, is known to be an effective and adjunctive method of disinfecting surfaces that may act as fomites (Qureshi Z, Yassin MH. Role of ultraviolet (UV) disinfection in infection control and environmental cleaning. Infectious Disorders Drug Targets. 2013 Jun;13(3):191-195). UVC light carries the highest photo-energy within the UV spectrum. UVC has a direct physical impact on the cellular genetic material (DNA), promoting cross-linking, hydrolysation, splitting and dimerization; all ultimately contributing to the halt of DNA replication, and thus inhibiting microbial cell division (Casini B, Tuvo B, Cristina ML, Spagnolo AM, Totaro M, Baggiani A, Privitera GP. Evaluation of an Ultraviolet C (UVC) Light-Emitting Device for Disinfection of High Touch Surfaces in Hospital Critical Areas. Int J Environ Res Public Health. 2019 Sep 24;16(19):3572). Other wavelengths of UV have also been investigated in terms of antimicrobial efficacy, and have been suggested to induce DNA-damage thus achieving antimicrobial efficacy (Hu, J., & Quek, P. H. (2008). Effects of UV radiation on photolyase and implications with regards to photoreactivation following low- and medium-pressure UV disinfection. Applied and environmental microbiology, 74(1), 327-328. https://doi.org/10.1128/AEM.00013-07.

The ultra violet light source may emit UV radiation between 100 and 280nm, in particular between 200 and 280nm, more particularly between 220 and 280nm, yet more particularly between 240 and 280nm, yet more particularly between 255 and 265nm, for example 260nm.

Additionally or alternatively, the ultra violet light source, or a separate UV light source may emit UV radiation having a wavelength of 280-315nm.

Additionally or alternatively, the ultra violet light source, or a separate UV light source may emit UV radiation having a wavelength of 315-400nm.

The degree of inactivation by ultraviolet radiation is directly related to the UV dose applied. The dosage, a product of UV light intensity and exposure time, is usually measured in millijoules per square centimetre ($mJs/cm^2$), or equivalently as milliwatt seconds per square centimetre ($mW \cdot s/cm^2$). Minimum dosages for a 90% kill of most bacteria and viruses typically range between 1 and 12 $mJs/cm^2$ (Hu, J., & Quek, P. H. (2008). Effects of UV radiation on photolyase and implications with regards to photoreactivation following low- and medium-pressure UV disinfection. Applied and environmental microbiology, 74(1), 327-328. https://doi.org/10.1128/AEM.00013-07. However, a higher dose of ~6J/$cm^2$ has been suggested to combat fungal pathogens (Dai, T., Kharkwal, G. B., Zhao, J., St Denis, T. G., Wu, Q., Xia, Y.,

Huang, L., Sharma, S. K., d'Enfert, C., & Hamblin, M. R. (2011). Ultraviolet-C light for treatment of Candida albicans burn infection in mice. Photochemistry and photobiology, 87(2), 342-349. https://doi.org/10.1111/j.1751-1097.2011.00886.x).

A liquid dispenser according to the preamble of claim 1 is known from CN 107 773 041 A.

In one embodiment of the present invention, the ultraviolet light source may emit a broad spectrum of ultraviolet radiation and/or emit variable wavelengths of UV radiation. The different UV wavelengths may be emitted concurrently or sequentially. In a further embodiment, the UV light source is operatively connected to a controller and the UV light source is controlled by the controller to emit pre-determined wavelengths of UV light for pre-determined intervals.

In an embodiment of the present invention, the door is located between a pair of opposing channels wherein opposing side edge portions of the door are located within a respective one of the channels. The door is free to move between the open and closed configurations within the channels.

It is an advantage of such an arrangement that, when the door is in the closed configuration, undesirable emission of UV at the edges of the door is prevented.

According to the invention, the dispenser further includes a controller and the movement of the door between its open and closed configurations is controlled by the controller. The use of a controller to control the opening and closing of the door allows for a "hands-free" opening and closing of the door.

It is an advantage that the controller may control the door to allow disinfection of the dispensing chamber and prevent cross-contamination, which, for example, may result from airborne contamination or droplet contamination, in particular by a user who has respiratory symptoms (e.g., coughing or sneezing) and/or has direct contact with the dispensing chamber.

Optionally, the controller may further control the operation of the disinfection system. Thus, the disinfection system may be controlled to operate only when the door is in its closed configuration. This prevents the potentially dangerous emission of UV light when the door is in its open configuration.

It is a further advantage of this embodiment that the controller controls the door and disinfection system and may provide automatic disinfection of the dispensing chamber when the door is closed.

The liquid dispensing apparatus may include a valve and/or a pump in addition to the dispensing port. The flow of liquid through the dispensing port may be controlled by the valve and/or pump. For example, when the valve is closed, the flow of liquid through the dispensing port may be prevented. Similarly, in embodiments in which the dispensing apparatus includes a pump, the flow of liquid may be controlled by the pump. For example, when the pump is energised, liquid may be permitted to flow through the dispensing port and when the pump is inactive, liquid may be prevented from flowing through the dispensing port, e.g. through the action of gravity. Suitably, the liquid to be dispensed is pressurised (e.g. mains pressure water) and the liquid dispensing apparatus includes one or more valves to control the flow of the liquid through a dispensing port of the liquid dispensing apparatus.

In one embodiment, the dispensing apparatus comprises more than one dispensing port, in particular two or three dispensing ports. In a further embodiment, the dispensing apparatus includes a valve and/or pump in addition to each dispensing port.

The or each valve and/or pump may be manually controlled or they may be automatically controlled via a controller. The controller that controls the operation of the valve and/or the operation of the pump may be the same controller as discussed above or it may be separate controller. In embodiments in which the valve and/or pump is manually controlled, this may be in a "hands-free" manner, for example, via one or more operative pedals.

In certain embodiments of the invention, the or each operative pedal may be connected to liquid dispensing apparatus via a controller.

The skilled person will appreciate that controlling the operation of the liquid dispensing apparatus automatically via a controller or manually in a "hands-free" manner allows for a hands-free operation of the liquid dispenser, which in turn limits the potential for contamination of the dispenser.

In a further embodiment of the invention, the dispenser includes a proximity sensor and the door is controlled to move from its closed configuration to its open configuration in response to signals from the proximity sensor and/or the operation of the liquid dispensing apparatus is controlled via signals from the proximity sensor. The proximity sensor permits a hands-free opening of the door and/or a hands-free operation of the liquid dispensing apparatus.

Suitably, the proximity sensor may be operatively connected to a controller and the movement of the door between its open and closed configurations and/or the operation of the liquid dispensing apparatus may be controlled via the controller. Thus, the controller may receive an input signal from the proximity sensor and it may transmit a corresponding control signal to the door and/or liquid dispensing apparatus in response to the input signal from the proximity sensor.

The proximity sensor of the present invention may be any type of proximity sensor, for example an ultrasonic proximity sensor.

In a further embodiment of the invention, the dispenser includes at least one cup sensor which is located within the dispensing chamber and which senses the height of a cup located within the dispensing chamber. Suitably, the cup sensor may be operatively connected to a controller and the controller controls the volume of water that is dispensed in accordance with the size of the cup located within the dispensing chamber. The cup sensor of the present invention may be any type of cup sensor, for example an ultrasonic cup sensor.

In a further embodiment, the dispensing chamber comprises more than one cup sensors, in particular two cup sensors.

In a further embodiment of the invention, the dispenser includes at least one foreign object sensor which is located within the dispensing chamber and which senses the presence of a foreign object within the dispensing chamber, such as a hand. Suitably, the foreign object sensor is operatively connected to the controller and the controller prevents the movement of the door to its closed configuration if a foreign object is sensed by the foreign object sensor within the dispensing chamber. The foreign object sensor of the present invention may be any type of foreign object sensor, for example an ultrasonic foreign object sensor.

In a further embodiment, the dispensing chamber comprises more than one foreign object sensors, in particular two foreign object sensors.

The skilled person will appreciate that the door may include an electric motor which drives it to open and close.

As noted hereinabove, the liquid dispenser may include one or more pedals. Thus, the dispenser may include a first control pedal, wherein the first control pedal is operatively connected to the door and/or the liquid dispensing apparatus. The first control pedal may be mechanically coupled to the door and/or the liquid dispensing apparatus. In such embodiments, the movement of the pedal may result in a corresponding opening of the door and/or a corresponding movement of a valve. Additionally or alternatively, the pedal may be electrically connected to a controller such that movement of the pedal by a user is translated into electrical signals which are received by the controller, and the controller transmits corresponding control signals to the door (e.g. to an electric motor which is arranged to move the door) and/or to the dispensing apparatus (e.g. to an electric motor which controls the position of a valve or to an electric pump).

It will be appreciated that the disinfection system may be configured in an inactive mode when the door is in its open configuration. In this way, UV exposure externally of the dispensing chamber may be prevented. Accordingly, the pedal may be directly or indirectly connected to the disinfection system such that operation of the pedal configures the disinfection system in an inactive state or mode.

In an embodiment of the invention, the door and the disinfection system are connected such that the disinfection system only operates when the door is in its closed configuration.

In a further embodiment of the invention, the dispenser includes a second control pedal, wherein the second control pedal is operatively connected to the liquid dispensing apparatus. In such an embodiment, the first pedal may operate the door and/or it may operate the liquid dispensing apparatus. Where the first pedal operates the door, the second pedal may operate the liquid dispensing apparatus. Alternatively, where the first pedal operates the liquid dispensing apparatus, it may operate a first part of the liquid dispensing apparatus and the second pedal may operate a second part of the liquid dispensing apparatus. For example, the liquid dispensing apparatus may be configured to dispense a heated liquid and a non-heated liquid (e.g. a liquid at ambient temperature or a cooled liquid), in which case, the first pedal may operate the liquid dispensing apparatus to dispense a heated liquid and the second pedal may operate the liquid dispensing apparatus to dispense a non-heated liquid.

In an embodiment of the invention, the liquid dispenser includes a housing which houses one or more of the components of the dispenser, for example, the dispensing chamber. The housing suitably defines a housing footprint. In other words, it defines an area of a substrate which the housing occupies. Optionally, the or each pedal of the liquid dispenser is recessed within the housing such that the pedals are located entirely within the footprint of the housing. Thus, the pedals do not extend beyond the footprint of the housing. This has the advantage that the pedals may be operated by a user, but do not present a trip hazard for people walking close to the liquid dispenser. Furthermore, this arrangement also prevents or minimises the accidental operation of the pedals.

The disinfection system may include a second component in addition to the ultra violet light source. For example, it may include a source of a disinfection fluid, for example a disinfecting gas (e.g. ozone or chlorine) and/or a disinfecting liquid which may be discharged into the dispensing chamber when the door is in its closed configuration.

Furthermore, the disinfection system may include two or more separate ultra violet light sources. For example, the apparatus may include a first UV light source which emits UV light when the door is in its closed configuration and a second UV light source which emits UV light periodically or according to a predetermined algorithm. The first UV light source may emit UV light with a first intensity and the second UV light source may emit UV light with a second intensity. Suitably, the second intensity is higher than the first intensity. In such arrangements, the first UV light source dispensing chamber at a "base" level; and the second UV light may provide a burst of relatively high intensity/power UV light energy to ensure complete sterilisation of the dispensing chamber at pre-determined intervals. This removes or inactivates at least some of the more resistant microorganisms. Such an arrangement provides an optimal balance between sterilisation of the dispensing chamber and energy consumption of the apparatus.

In one embodiment of the invention, the door remains in an open configuration while the liquid is dispensed. However, in an alternative embodiment, the door may be controlled to open upon demand to permit a user to place a cup or other liquid container within the dispensing chamber. Once the cup/container is detected within the dispensing chamber, the door may be controlled such that it moves to its closed configuration prior to the liquid being dispensed. In this way, the dispensing chamber and the cup/container may be disinfected before, during and/or after the liquid is dispensed into the cup/container. Thus, the dispensing step may be controlled such that it is carried out only with the door in its closed configuration. Once the liquid has been dispensed and the dispensing chamber and cup/container has been disinfected, the door may re-open such that the user is able to retrieve the filled and disinfected cup/container. Accordingly, the controller may be programmed to open the door; sense a container located within the dispensing chamber; close the door; disinfect the dispensing chamber and the container before, during and/or after the liquid is dispensed into the container; and re-open the door once the dispensing and/or disinfection step is complete.

The liquid dispenser according to the first aspect of the invention may be configured for retro-fitting to an existing liquid dispensing system. Alternatively, it may form part of a novel liquid dispensing system.

In this context, the term 'for retro-fitting' means suitable for modifying equipment that is already in service using parts developed or made available after the time of original manufacture. In particular, in the context of the present invention, this term means suitable for modifying an existing liquid dispensing system to provide a hands-free liquid dispensing system with ultra violet disinfection to minimise cross-contamination by infectious agents.

Thus, according to a second aspect of the invention, there is provided a liquid dispensing system including a liquid dispenser according to the first aspect of the invention as defined anywhere herein, and a source of liquid to be dispensed, wherein the liquid dispensing apparatus is fluidly coupled to the source of the liquid to be dispensed.

The liquid to be dispensed may be water and the source of the water may be a detachable reservoir or a mains water source.

In one embodiment, the liquid dispensing system comprises one or more water filters which filter the water prior to being dispensed. In this embodiment, the or each filter removes impurities and/or infectious agents from the water before it is dispensed.

The liquid dispensing system may further include a heater to heat the liquid to be dispensed. Additionally or alternatively, the liquid dispensing system may include a chiller to cool the liquid to be dispensed.

In embodiments in which the liquid dispenser includes one or more electric motors, or the liquid dispensing system includes a heater and/or chiller, the liquid dispensing system suitably also includes a source of electrical energy. For example, it may include an electrical plug for connection to a mains electricity system and/or it may include a rechargeable battery system and/or it may include a photovoltaic cell.

In a further embodiment, the liquid dispensing system includes a display. Such a display may instruct a user as to the operation of the dispensing system and/or it may provide information relating to the liquid to be dispensed, e.g. the temperature of the liquid, the disinfection status and/or the remaining volume of liquid stored in a liquid reservoir.

In a third aspect, the present invention provides a method of manufacturing a hands-free liquid dispensing system with ultraviolet disinfection to minimise cross-contamination by infectious agents, which comprises the step of fluidly coupling a liquid dispenser according to the present invention, which is configured for retro-fitting, to a liquid dispensing system.

An embodiment of the invention will now be described, by way of example only, with reference to the accompanying drawings in which:

Figure 1 shows a cross sectional view of a liquid dispensing system according to an embodiment of the second aspect of the invention;
Figure 1a shows an enlarged view of a portion of the door shown in Figure 1;
Figure 1b shows an enlarged view of a cup sensor shown in Figure 1;
Figure 1c shows an enlarged view of a safety sensor shown in Figure 1;
Figure 1d shows an enlarged view of a water filter shown in Figure 1;
Figure 2 shows a front elevational view of the liquid dispensing system shown in Figure 1;
Figure 2a shows an enlarged view of the pedals shown in Figure 2;
Figure 2b shows an enlarged view of an ultra violet light source shown in Figure 2;
Figure 2c shows an enlarged view of a display screen shown in Figure 2; and
Figure 3 shows a retrofit liquid dispenser according to a second embodiment which is connected to an existing liquid dispensing system.

For the avoidance of doubt, the skilled person will appreciate that in this specification, the terms "up", "down", "front", "rear", "upper", "lower", "width", etc. refer to the orientation of the components as found in the example when installed for normal use as shown in the Figures.

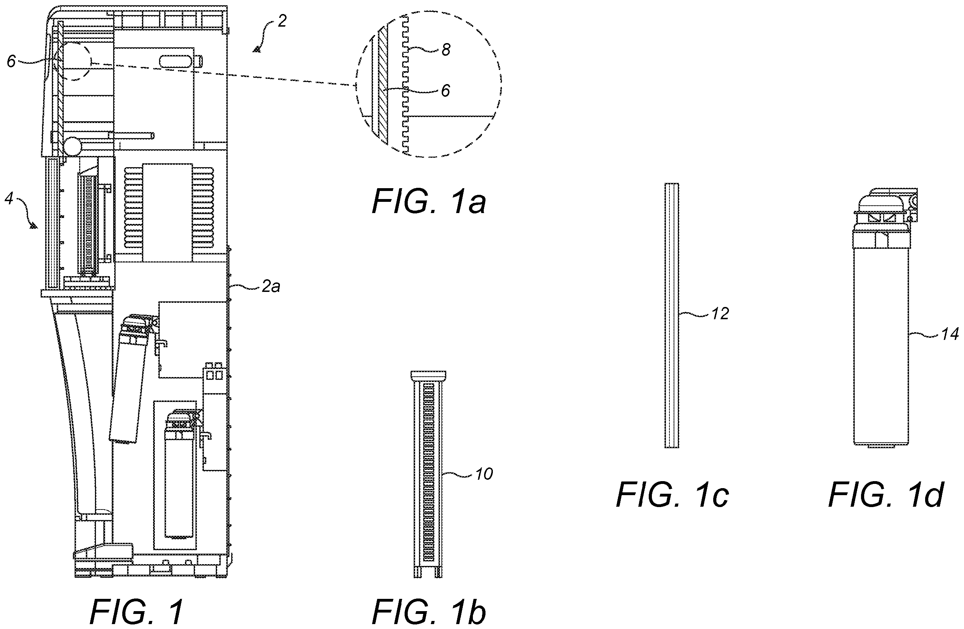

Figure 1 shows a liquid dispensing system 2 which is a "hands-free" system and which automatically disinfects a dispensing chamber 4 after each use. The liquid dispensing system 2 includes a housing 2a within which the components which form the liquid dispensing system 2 are housed. The housing 2a defines the dispensing chamber 4.

In this embodiment, the liquid dispensing system 2 is a water/liquid dispenser which is capable of dispensing both chilled water/liquid and hot water/liquid, as desired.

The dispensing chamber 4 includes a door 6, which has an open configuration in which access to the dispensing chamber 4 is permitted and a closed configuration in which access to the dispensing chamber is prevented.

As the dispensing chamber 4 is disinfected using ultra violet light, the door 6 is made from an opaque material to prevent UV light from being emitted through the door 6. In order to minimise UV light escaping from between the side edge portions of the door 6 and the sides of the dispensing chamber 4, the side wall portions of the dispensing chamber 4 carry a pair of opposed U-shaped tracks. Each side edge portion of the door 6 is located within a respective one of the U-shaped tracks, wherein the door is able to move within the tracks vertically upwards and downwards relative to the side wall portions of the chamber 4. This arrangement prevents UV light from escaping from the dispensing chamber between the side edge portions of the door 6.

As shown in Figure 1a, the door 6 includes a linear array of teeth 8 on its rear surface. An electric motor (not shown) is able to drive the door 6 to raise and lower via a gear (not shown) carried by a drive shaft of the motor, which is in a meshed engagement with the teeth 8. Such an arrangement is well known to the person skilled in the art and as such needs not be described in detail herein.

It will be appreciated that the door 6 is in its open configuration when raised, as this permits access to the front of the dispensing chamber 4. Similarly, the door 6 is in its closed configuration when it is lowered, as in this configuration, the front of the dispensing chamber 4 is closed by the door 6.

A proximity sensor (not shown) may be provided which causes the door to move to its open configuration when the proximity sensor senses a user close to the system 2.

Figure 1b shows a cup sensor 10 which is located within the dispensing chamber 4 and which senses the height of a cup located within the dispensing chamber 4. The cup sensor 10 is electrically connected to a controller (not shown) and the controller controls the volume of water that is dispensed by the system 2 in accordance with the size of the cup located within the dispensing chamber 4.

Figure 1c shows a foreign object sensor 12. This sensor senses the presence of a foreign object within the dispensing chamber 4, such as a hand. The foreign object sensor 12 is also connected to the controller and the controller prevents the movement of the door 6 to its closed configuration if a foreign object is sensed by the foreign object sensor 12 within the dispensing chamber 4.

Figure 1d shows a water filter 14, which filters the water prior to being dispensed. In this embodiment, the system 2 is connected to a source of mains water and the filter 14 removes impurities and infectious agents from the mains water before it is dispensed.

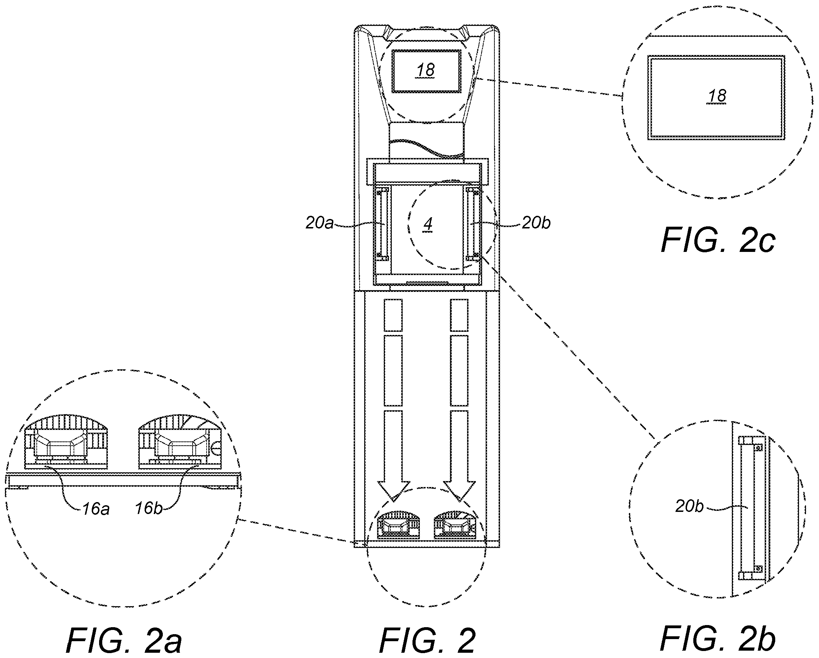

Figure 2 shows a front view of the system 2. The system 2 is operated via a pair of foot pedals 16a, 16b which are shown in more detail in Figure 2a. In this embodiment, the left pedal 16a is connected to an apparatus for dispensing chilled water. Thus, the left pedal 16a is connected to the controller. In an embodiment of the invention, when the controller senses an input from the left pedal 16a as a result of the left pedal 16a being depressed by a user, it sends a control signal to the door motor to move the door 6 to its open configuration. Alternatively, where a proximity sensor is provided and the door is moved to its open configuration in response to a signal from the proximity sensor, the controller senses the size of a cup located within the dispensing chamber 4 in response to operation of the left pedal 16a. If no cup is sensed in the dispensing chamber 4, the controller sends an alert signal to the user via an LED display 18 (shown in Figure 2c). The cup is then filled with chilled water according to an algorithm contained within the controller which calculates the volume of water to be dispensed based on the size of the cup sensed within the dispensing chamber 4. If the water is dispensed under the influence of gravity or from a pressurised source of water (e.g. a mains source of water), then the dispensing of water may be controlled via the controller sending a control signal to a valve such that the valve is opened for a pre-determined period of time. If the water is dispensed via a pump, then the dispensing of water may be controlled via the controller sending a control signal to the pump to operate for a pre-determined period of time. The chilled water dispensing apparatus includes a chiller which chills the water prior to being dispensed to a desired temperature.

The right pedal 16b is connected to an apparatus for dispensing heated water. This operates similar to the operation of the left pedal 16a, except that the heated water dispensing apparatus includes a water heater which heats the water prior to it being dispensed.

The heating and chilling of water prior to being dispensed is well known in the art. Accordingly, it is not necessary to describe in detail the heated water dispensing apparatus and the chilled water dispensing apparatus.

After the water has been dispensed, the foreign object sensor 12 senses if a foreign object is present in the dispensing chamber 4. If no foreign object is sensed, the foreign object sensor 12 sends a signal to the controller. Upon receipt of the signal from the foreign object sensor 12, the controller sends a control signal to the door motor, which in turn moves the door 6 to its closed configuration. Once the door is in its closed configuration, the controller sends a control signal to a pair of ultra violet light assemblies 20a, 20b (UV light assembly 20b is shown in more detail in Figure 2b) which are located on either side of the dispensing chamber 4. The ultra violet light assemblies 20a, 20b emit UV light into the closed dispensing chamber 4 which kills any infectious agents present within the dispensing chamber 4. The controller controls the UV light assemblies 20a, 20b to emit UV light for a pre-determined period of time.

The controller prevents the door motor from operating while the UV light assemblies 20a, 20b are emitting UV light.

When the UV light assemblies 20a, 20b have stopped emitting UV light, the controller permits operation of the door, either via the proximity sensor or via operation of one of the pedals 16a, 16b.

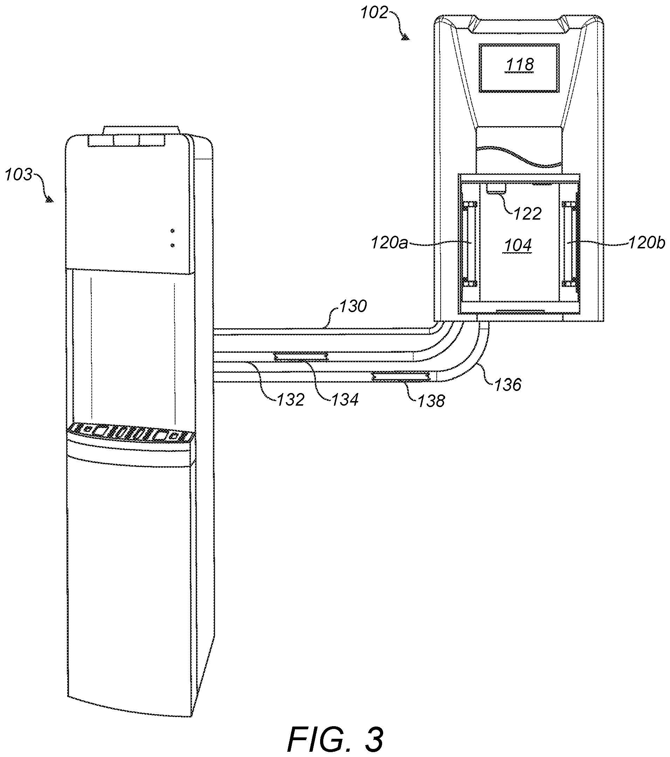

Figure 3 shows a second embodiment of the invention in which a liquid dispenser 102 has been retrofitted to an existing liquid dispensing system 103. In this embodiment, the liquid dispenser 102 is substantially identical to that described hereinabove in connection with the first embodiment shown in Figures 1 and 2. Thus, the liquid dispenser 102 defines a dispensing chamber 104 which may be closed via a door (not shown in Figure 3, but identical to the door 6 shown in Figure 1 and described above). The dispensing chamber 104 further includes a pair of opposed UV light emitters 120a, 120b, a cup sensor (not shown) and a foreign object sensor (not shown). Finally, the dispensing chamber includes a dispensing nozzle 122, which is coupled via a manifold (not shown) to a hot water conduit 130, an ambient water conduit 132 and a chilled water conduit 136.

The hot water conduit 130 is connected at its opposite end to a water heater (not shown) which is located within the existing liquid dispensing system 103 and which heats a source of mains water to a pre-determined temperature. Similarly, the ambient water conduit 132 is connected at its opposite end to an outlet for a mains source of water housed within the existing liquid dispensing system 103. Finally, the chilled water conduit 136 is connected at its opposite end to a chiller system housed within the existing liquid dispensing system 103 which chills a source of mains water to a pre-determined temperature.

In order to purify the ambient water and the chilled water dispensed from the liquid dispenser, the ambient water conduit 132 includes a filter 134 located within the conduit 132. Similarly, the chilled water conduit 136 includes a similar filter 138 located within the conduit 136. These filters are conventional filters for use with sources of ambient and/or chilled water for drinking. The hot water conduit 130 does not need such a filter, as the heated water contains fewer potential contaminants.

As with the liquid dispensing system 2, the liquid dispenser 102 includes an LED display screen 118 which provides information to a user.

Experimental

Experiments were carried out to determine the efficiency of UV irradiation of a chamber equivalent in size to a liquid dispensing chamber.

A TCID50 assay was employed which used Madin-Darby Canine Kidney (MDCK) cells as the tissue culture. The test virus was an influenza virus of the family Orthomyxoviridae, which is an enveloped, single-stranded segmented RNA virus.

The TCID50 is defined as the dilution of virus required to infect 50% of the inoculated cells, based on the production of an observable cytopathic effect (CPE).

The host cells are grown in confluent monolayers to which various dilutions of the virus are added. The virus replicates and the progeny virus that is released into the supernatant fluid is free to infect other cells. The CPE damage is allowed to develop, after which time the cells are stained or observed microscopically and the results (presence or absence of CPE) are recorded.

An Influenza virus titre of stock is calculated using the Reed and Muench method.

The TCID50 assay was used in this process to determine the titre of the influenza virus according to the protocol. The MDCK cells were cultured in 96 well plates at 90-100% confluency. One millilitre of virus suspension in a suitable container was placed in different positions within a test chamber and exposed to UV irradiation at room temperature for different periods of time. A control sample containing one millilitre of virus suspension was incubated at room temperature without UV irradiation as a positive control. At the end of the irradiation period, ten-fold dilutions from the recovered virus from the samples and the positive control were prepared using DMEM followed by infection of the MDCK cells at 37°C for one hour. The 96 well plates were then incubated for 72 hours at 37°C in a $CO_2$ incubator.

The virus suspension was irradiated in the chamber for 15s, 30s, 60s, 2 min, 5 min, 10 min and 15 min prior to recovery. The UV emitter was a UVC lamp which emits UV light with a wavelength of 260nm.

The TCID50 of the virus was calculated by the method of Reed and Muench using the following formula:

$$((\% \text{ above } 50\%)-50\%)/((\% \text{ above } 50\%)-(\% \text{ below } 50\%)) = \text{proportionate distance (PD)}$$

$$(\log \text{ dilution above } 50\%) + (PD \times \log \text{ dilution factor}) = \log \text{ TCID50}$$

The antimicrobial efficacy of UVC was also investigated against bacterial and fungal species; particularly S. *aureus* as a gram positive, *P. aeruginosa* as a gram negative, and *A. brasiliensis* as a fungal representative. These are common contaminants associated with moist environments, and human contact pathogens. Cultures of the representative strains were obtained, and either treated as negative control samples, or treatment, whereby microbial cultures were exposed to predetermined intervals of UVC light. The colony forming units (CFU) of growth were compared in samples exposed to UV, versus the initial microbial count, to obtain the log reduction in microbial load. These results are represented in Table 1. The calculated titre of the virus recovered from the samples and the log reduction of the test samples are also shown in Table 1.

Table 1: Microbial Log $_{10}$ Reduction Following UVC Exposure

|  | Viral Log Reduction | Gram Positive Log Reduction | Gram Negative Log Reduction | Fungal Log Reduction |
|---|---|---|---|---|
| 15 Second irradiation | 1 | 1 | 1 | No reduction |
| 30 second irradiation | 1.2 | 1 | 2 | 1 |
| 60 second irradiation | 1.9 | 2 | 2 | 1 |
| 2 min irradiation | 7.1 | 2 | 2 | 2 |
| 5 min irradiation | 7.1 | 2 | 2 | 2 |
| 10 min irradiation | 7.1 | 2 | 3 | 2 |
| 15 min irradiation | 7.1 | 3 | 3 | ~3 |

These results show that irradiation of the chamber for a period of time of at least 2 minutes is sufficient to inactivate the influenza virus.

By way of example only, suitable sanitation regimen using the liquid dispenser of the present invention are described in the following examples.

Example 1

Liquid dispenser of the present invention in use in a school:

| Time | Sanitation description | Notes |
|---|---|---|
| 06:00 am - 06:15 am | Continuous sanitation for 15 minutes | - |
| 06:15 am - 09:55 am | Sanitation for 15 seconds after each use | Students break: from 10:15 am to 10:30 am |
| 09:55 am - 10:00 am | Continuous sanitation for 5 minutes | Students break: from 10:15 am to 10:30 am |
| 10:00 am - 10:45 am | Sanitation for 15 seconds after each use | Students break: from 10:15 am to 10:30 am |
| 10:45 am - 10:50 am | Continuous sanitation for 5 minutes | - |
| 10:50 am - 11:40 am | Sanitation for 15 seconds after each use | - |
| 11:40 am - 11:45 am | Continuous sanitation for 5 minutes | Lunch break: from 12:00 pm to 12:55 pm |
| 11:45 am - 01:15 pm | Sanitation for 15 seconds after each use | Lunch break: from 12:00 pm to 12:55 pm |
| 01:15 pm - 01:20 pm | Continuous sanitation for 5 minutes | - |
| 01:20 pm - 02:50 pm | Sanitation for 15 seconds after each use | Students break: from 02:25 pm to 02:40 pm |
| 02:50 pm - 02:55 pm | Continuous sanitation for 5 minutes | Students break: from 02:25 pm to 02:40 pm |
| 02:55 pm - 04:30 pm | Sanitation for 15 seconds after each use | School day ends at 04:00 pm |
| 04:30 pm - 04:45 pm | Continuous sanitation for 15 minutes |  |

(continued)

| Time | Sanitation description | Notes |
|---|---|---|
| 04:30 pm - 06:00 am | Sanitation for 15 seconds after each use | When water dispensers are kept on and in use overnight |

**Example 2**

Liquid dispenser of the present invention in use in a common room in a university department

| Time | Sanitation description | Notes |
|---|---|---|
| 05:00 am - 05:15 am | Continuous sanitation for 15 minutes | |
| 05:15 am - 10:00 am | Sanitation for 15 seconds after each use | |
| 10:00 am - 10:05 am | Continuous sanitation for 5 minutes | |
| 10:05 am - 02:00 pm | Sanitation for 15 seconds after each use | |
| 02:00 pm - 02:05 pm | Continuous sanitation for 5 minutes | The common room is open for 24 hours/ day and 7 days/week. It is mainly used by research students and academic staff for tea/ lunch breaks and short meetings. If a person approaches the water dispenser during any of the 5-minute sanitation periods, the sanitation will be paused, dispensing will take place and the sanitation will be resumed afterwards. |
| 02:05 pm - 06:00 pm | Sanitation for 15 seconds after each use | |
| 06:00 pm - 06:05 pm | Continuous sanitation for 5 minutes | |
| 06:05 pm - 10:00 pm | Sanitation for 15 seconds after each use | |
| 10:00 pm - 10:15 pm | Continuous sanitation for 15 minutes | |
| 10:15 pm - 05:00 am | Sanitation for 15 seconds after each use | |

**Claims**

1.    A liquid dispenser comprising a dispensing chamber (4), wherein the dispensing chamber (4) includes a liquid dispensing apparatus, wherein the liquid dispensing apparatus includes a dispensing port; a closable door (6) which has an open configuration in which access to the chamber (4) is permitted, and a closed configuration in which access to the chamber (4) is prevented; and a disinfection system comprising an ultra violet light source (20), wherein the chamber (4) is disinfected via the disinfection system when the door (6) is in its closed configuration, **characterised in that** the dispenser further includes a controller and the movement of the door (6) between its open and closed configurations is controlled by the controller.

2. A liquid dispenser according to Claim 1, wherein the controller further controls the operation of the disinfection system.

3. A liquid dispenser according to Claim 1 or Claim 2, wherein the liquid dispensing port includes a valve and the valve is operated via control signals from the controller.

4. A liquid dispenser according to any of Claims 1 to 3, wherein the liquid dispensing apparatus includes a liquid pump; and operation of the liquid pump is controlled via control signals from the controller.

5. A liquid dispenser according to any of Claims 1 to 4, wherein the dispenser includes a proximity sensor and the door is controlled to move from its closed configuration to its open configuration in response to signals from the proximity sensor and/or the operation of the liquid dispensing apparatus is controlled via signals from the proximity sensor, optionally wherein the proximity sensor is operatively connected to the controller, and the movement of the door and operation of the liquid dispensing apparatus is controlled by the controller, optionally wherein the proximity sensor is an ultrasonic sensor.

6. A liquid dispenser according to any of Claims 1 to 5, wherein the dispenser includes a first control pedal (16a), wherein the first control pedal (16a) is operatively connected to the door and/or the liquid dispensing apparatus, optionally via a controller,, and optionally wherein the dispenser further includes a second control pedal (16b), wherein the optional second control pedal (16b) is operatively connected to the liquid dispensing apparatus.

7. A liquid dispenser according to Claim 6, wherein the liquid dispenser includes a housing (2a), the housing defines a housing footprint, the or each pedal (16a/16b) is recessed within the housing (2a) such that the or each pedal (16a/16b) does not extend beyond the housing footprint.

8. A liquid dispenser according to any of Claims 1 to 7, wherein the liquid dispensing apparatus is fluidly coupled to a source of chilled or ambient temperature liquid and/or heated liquid.

9. A liquid dispenser according to any of Claims 1 to 8, wherein the liquid dispenser dispenses water.

10. A liquid dispenser according to any of Claims 1 to 9, wherein the dispenser includes a display (18).

11. A liquid dispenser according to any of Claims 1 to 10, wherein the door is located between a pair of opposing channels, wherein opposing side edge portions of the door are located within a respective one of the channels.

12. A liquid dispenser according to any of Claims 1 to 11, wherein the controller further controls the operation of the disinfection system; and the controller is programmed to (i) open the door (6) on demand, whereby the dispensing chamber is capable of receiving a container therein; (ii) close the door (6) when a container is disposed within the dispensing chamber; (iii) disinfect the dispensing chamber and the container disposed therein before, during and/or after a liquid has been dispensed into the container; and (iv) re-open the door (6) when after the liquid has been dispensed and the dispensing chamber and container have been disinfected, optionally wherein the chamber (4) further includes a container sensor (10) which is connected to the controller.

13. A liquid dispenser according to Claim 12, wherein the liquid dispensing port includes a valve and the valve is operated via control signals from the controller and/or the liquid dispensing apparatus includes a liquid pump; and operation of the liquid pump is controlled via control signals from the controller.

14. A liquid dispensing system (2) including a liquid dispenser according to any of Claims 1 to 13 and a source of liquid to be dispensed, wherein the liquid dispensing apparatus is fluidly coupled to the source of the liquid to be dispensed.

15. A method of manufacturing a hands-free liquid dispensing system with ultraviolet disinfection to minimise cross-contamination by infectious agents, which comprises the step of fluidly coupling a liquid dispenser (102) according to any of claims 1 o 13, which is configured for retro-fitting, to a liquid dispensing system (103).

**Patentansprüche**

1.  Flüssigkeitsspender, umfassend eine Abgabekammer (4), wobei die Abgabekammer (4) eine Flüssigkeitsabgabevorrichtung beinhaltet, wobei die Flüssigkeitsabgabevorrichtung eine Abgabeöffnung beinhaltet; eine verschließbare Tür (6), die eine offene Konfiguration aufweist, in der Zugang zu der Kammer (4) gestattet ist, und eine geschlossene Konfiguration, in der Zugang zu der Kammer (4) verhindert wird; und ein Desinfektionssystem umfassend eine Ultraviolettlichtquelle (20), wobei die Kammer (4) mittels des Desinfektionssystems desinfiziert wird, wenn die Tür (6) in ihrer geschlossenen Konfiguration ist, **dadurch gekennzeichnet, dass** der Spender ferner eine Steuerung umfasst und die Bewegung der Tür (6) zwischen ihrer offenen und geschlossenen Konfiguration von der Steuerung gesteuert wird.

2.  Flüssigkeitsspender nach Anspruch 1, wobei die Steuerung ferner den Betrieb des Desinfektionssystems steuert.

3.  Flüssigkeitsspender nach Anspruch 1 oder Anspruch 2, wobei die Flüssigkeitsabgabeöffnung ein Ventil beinhaltet und das Ventil über Signale aus der Steuerung betätigt wird.

4.  Flüssigkeitsspender nach einem der Ansprüche 1 bis 3, wobei die Flüssigkeitsabgabevorrichtung eine Flüssigkeitspumpe beinhaltet; und der Betrieb der Flüssigkeitspumpe über Steuersignale aus der Steuerung gesteuert wird.

5.  Flüssigkeitsspender nach einem der Ansprüche 1 bis 4, wobei der Spender einen Näherungssensor beinhaltet und die Tür gesteuert wird, um sich als Reaktion auf Signale aus dem Näherungssensor von ihrer geschlossenen Konfiguration in ihre offene Konfiguration zu bewegen, und/oder der Betrieb der Flüssigkeitsabgabevorrichtung über Signale aus dem Näherungssensor gesteuert wird, optional, wobei der Näherungssensor operativ mit der Steuerung verbunden ist und die Bewegung der Tür und der Betrieb der Flüssigkeitsabgabevorrichtung von der Steuerung gesteuert werden, optional, wobei der Näherungssensor ein Ultraschallsensor ist.

6.  Flüssigkeitsspender nach einem der Ansprüche 1 bis 5, wobei der Spender ein erstes Steuerpedal (16a) beinhaltet, wobei das erste Steuerpedal (16a) operativ mit der Tür und/oder der Flüssigkeitsabgabevorrichtung verbunden ist, optional über eine Steuerung, und optional, wobei der Spender ferner ein zweites Steuerpedal (16b) beinhaltet, wobei das optionale zweite Steuerpedal (16b) operativ mit der Flüssigkeitsabgabevorrichtung verbunden ist.

7.  Flüssigkeitsspender nach Anspruch 6, wobei der Flüssigkeitsspender ein Gehäuse (2a) beinhaltet, das Gehäuse ein Gehäuseprofil definiert, das oder jedes Pedal (16a/16b) innerhalb des Gehäuses (2a) so eingelassen ist, dass sich das oder jedes Pedal (16a/16b) nicht über das Gehäuseprofil hinaus erstreckt.

8.  Flüssigkeitsspender nach einem der Ansprüche 1 bis 7, wobei die Flüssigkeitsabgabevorrichtung fluidisch mit einer Quelle von gekühlter oder Umgebungstemperatur aufweisender Flüssigkeit und/oder erhitzter Flüssigkeit gekoppelt ist.

9.  Flüssigkeitsspender nach einem der Ansprüche 1 bis 8, wobei der Flüssigkeitsspender Wasser abgibt.

10. Flüssigkeitsspender nach einem der Ansprüche 1 bis 9, wobei der Spender eine Anzeige (18) beinhaltet.

11. Flüssigkeitsspender nach einem der Ansprüche 1 bis 10, wobei die Tür zwischen einem Paar gegenüberliegender Kanäle befindlich ist, wobei gegenüberliegende Seitenkantenabschnitte der Tür innerhalb eines jeweiligen der Kanäle befindlich sind.

12. Flüssigkeitsspender nach einem der Ansprüche 1 bis 11, wobei die Steuerung ferner den Betrieb des Desinfektionssystems steuert; und die Steuerung programmiert ist, um (i) die Tür (6) auf Anforderung zu öffnen, wodurch die Abgabekammer einen Behälter darin aufnehmen kann; (ii) die Tür (6) zu schließen, wenn ein Behälter innerhalb der Abgabekammer angeordnet ist; (iii) die Abgabekammer und den darin angeordneten Behälter zu desinfizieren, bevor, während und/oder nachdem eine Flüssigkeit in den Behälter abgegeben worden ist; und (iv) die Tür (6) wieder zu öffnen, nachdem die Flüssigkeit abgegeben worden ist und die Abgabekammer und der Behälter desinfiziert worden sind, optional, wobei die Kammer (4) ferner einen Behältersensor (10) beinhaltet, der mit der Steuerung verbunden ist.

13. Flüssigkeitsspender nach Anspruch 12, wobei die Flüssigkeitsabgabeöffnung ein Ventil beinhaltet und das Ventil über Steuersignale aus der Steuerung betätigt wird und/oder die Flüssigkeitsabgabevorrichtung eine Flüssigkeitspumpe beinhaltet; und der Betrieb der Flüssigkeitspumpe über Steuersignale aus der Steuereinheit gesteuert wird.

14. Flüssigkeitsabgabesystem (2) beinhaltend einen Flüssigkeitsspender nach einem der Ansprüche 1 bis 13 und eine Quelle von abzugebender Flüssigkeit, wobei die Flüssigkeitsabgabevorrichtung fluidisch mit der Quelle der abzugebenden Flüssigkeit verbunden ist.

15. Verfahren zur Herstellung eines Freihand-Flüssigkeitsabgabesystems mit Ultraviolettdesinfektion zur Minimierung der Kreuzkontamination durch infektiöse Agenzien, das den Schritt des fluidischen Koppelns eines Flüssigkeitsspenders (102) nach einem der Ansprüche 1 bis 13, der zur Nachrüstung konfiguriert ist, an ein Flüssigkeitsabgabesystem (103) umfasst.

**Revendications**

1. Un distributeur de liquide comprenant une chambre de distribution (4), la chambre de distribution (4) comprenant un appareil de distribution de liquide, l'appareil de distribution de liquide comprenant un orifice de distribution ; une porte fermable (6) ayant une configuration ouverte dans laquelle l'accès à la chambre (4) est permis, et une configuration fermée dans laquelle l'accès à la chambre (4) est empêché ; et un système de désinfection comprenant une source de lumière ultraviolette (20), la chambre (4) étant désinfectée par l'intermédiaire du système de désinfection quand la porte (6) est en configuration fermée, **caractérisé par le fait que** le distributeur comprend en sus un contrôleur et le mouvement de la porte (6) entre ses configurations ouverte et fermée est commandé par le contrôleur.

2. Un distributeur de liquide selon la revendication 1, dans lequel le contrôleur commande en sus le fonctionnement du système de désinfection.

3. Un distributeur de liquide selon la revendication 1 ou la revendication 2, dans lequel l'orifice de distribution de liquide comprend une vanne et la vanne est actionnée par l'intermédiaire de signaux de commande venant du contrôleur.

4. Un distributeur de liquide selon une quelconque des revendications 1 à 3, dans lequel l'appareil de distribution de liquide comprend une pompe à liquide, le fonctionnement de la pompe à liquide étant commandé par l'intermédiaire de signaux de commande venant du contrôleur.

5. Un distributeur de liquide selon une quelconque des revendications 1 à 4, dans lequel le distributeur comprend un capteur de proximité et la porte est commandée pour passer de sa configuration fermée à sa configuration ouverte en réponse à des signaux du capteur de proximité et/ou dans lequel le fonctionnement de l'appareil de distribution de liquide est commandé par l'intermédiaire de signaux venant du capteur de proximité, le capteur de proximité étant éventuellement raccordé fonctionnellement au contrôleur, le mouvement de la porte et le fonctionnement de l'appareil de distribution de liquide étant commandés par le contrôleur, le capteur de proximité étant éventuellement un capteur à ultrasons.

6. Un distributeur de liquide selon une quelconque des revendications 1 à 5, dans lequel le distributeur comprend une première pédale de commande (16a), la première pédale de commande (16a) étant raccordée fonctionnellement à la porte et/ou à l'appareil de distribution de liquide, éventuellement par l'intermédiaire d'un contrôleur, le distributeur comprenant éventuellement une deuxième pédale de commande (16b) , l'éventuelle deuxième pédale de commande (16b) étant raccordée fonctionnellement à l'appareil de distribution de liquide.

7. Un distributeur de liquide selon la revendication 6, le distributeur de liquide comprenant un boîtier (2a), le boîtier définissant une empreinte de boîtier, la ou chaque pédale (16a/16b) étant encastrée à l'intérieur du boîtier (2a) de manière à ce la ou chaque pédale (16a/16b) ne dépasse pas l'empreinte du boîtier.

8. Un distributeur de liquide selon une quelconque des revendications 1 à 7, dans lequel l'appareil de distribution de liquide est couplé fluidiquement à une source de liquide réfrigéré ou à température ambiante et/ou de liquide chauffé.

9. Un distributeur de liquide selon une quelconque des revendications 1 à 8, le distributeur de liquide distribuant de l'eau.

10. Un distributeur de liquide selon une quelconque des revendications 1 à 9, dans lequel le distributeur comprend un affichage.

**11.** Un distributeur de liquide selon une quelconque des revendications 1 à 10, dans lequel la porte est située entre une paire de canaux opposés, des parties de bord latéral opposées de la porte étant respectivement situées à l'intérieur d'un des canaux.

**12.** Un distributeur de liquide selon une quelconque des revendications 1 à 11, dans lequel le contrôleur commande en sus le fonctionnement du système de désinfection ; et le contrôleur est programmé pour (i) ouvrir la porte (6) à la demande, grâce à quoi la chambre de distribution est capable de recevoir un récipient à l'intérieur ; (ii) fermer la porte (6) quand un récipient est placé à l'intérieur de la chambre de distribution ; (iii) désinfecter la chambre de distribution et le récipient placé à l'intérieur avant, pendant et/ou après qu'un liquide a été distribué dans le récipient ; et (iv) rouvrir la porte (6) une fois que le liquide a été distribué et que la chambre de distribution et le récipient ont été désinfectés, la chambre (4) comprenant éventuellement un capteur de récipient (10) raccordé au contrôleur.

**13.** Un distributeur de liquide selon la revendication 12, dans lequel l'orifice de distribution de liquide comprend une vanne et la vanne est actionnée par l'intermédiaire de signaux de commande venant du contrôleur et/ou l'appareil de distribution de liquide comprend une pompe à liquide ; et le fonctionnement de la pompe à liquide est commandé par l'intermédiaire de signaux de commande venant du contrôleur.

**14.** Un système de distribution de liquide (2) comprenant un distributeur de liquide selon une quelconque des revendications 1 à 13, et une source de liquide à distribuer, l'appareil de distribution de liquide étant couplé fluidiquement à la source du liquide à distribuer.

**15.** Un procédé de fabrication d'un système de distribution de liquide mains libres avec désinfection aux ultraviolets pour minimiser la contamination croisée par des agents infectieux, qui comprend l'étape consistant à coupler fluidiquement un distributeur de liquide (102) selon une quelconque des revendications 1 à 13, qui est configuré pour modification ultérieure, à un système de distribution de liquide (103).

FIG. 1d

FIG. 1c

FIG. 1a

FIG. 1b

FIG. 1

FIG. 2a

FIG. 2

FIG. 2b

FIG. 2c

EP 4 251 556 B1

**FIG. 3**

## REFERENCES CITED IN THE DESCRIPTION

### Patent documents cited in the description

- CN 107773041 A

### Non-patent literature cited in the description

- **QURESHI Z ; YASSIN MH.** Role of ultraviolet (UV) disinfection in infection control and environmental cleaning. *Infectious Disorders Drug Targets,* June 2013, vol. 13 (3), 191-195
- **CASINI B ; TUVO B ; CRISTINA ML ; SPAGNOLO AM ; TOTARO M ; BAGGIANI A ; PRIVITERA GP.** Evaluation of an Ultraviolet C (UVC) Light-Emitting Device for Disinfection of High Touch Surfaces in Hospital Critical Areas. *Int J Environ Res Public Health,* 24 September 2019, vol. 16 (19), 3572
- **HU, J. ; QUEK, P. H.** Effects of UV radiation on photolyase and implications with regards to photoreactivation following low- and medium-pressure UV disinfection. *Applied and environmental microbiology,* 2008, vol. 74 (1), 327-328, https://doi.org/10.1128/AEM.00013-07
- **DAI, T. ; KHARKWAL, G. B. ; ZHAO, J. ; ST DENIS, T. G. ; WU, Q. ; XIA, Y. ; HUANG, L. ; SHARMA, S. K. ; D'ENFERT, C. ; HAMBLIN, M. R.** Ultraviolet-C light for treatment of Candida albicans burn infection in mice. *Photochemistry and photobiology,* 2011, vol. 87 (2), 342-349, https://doi.org/10.1111/j.1751-1097.2011.00886.x